# FASCICULE DE BREVET EUROPEEN

(11) **EP 0 665 732 B1**
(45) Date de publication et mention de la délivrance du brevet: **18.12.1996**
(21) Numéro de dépôt: 93922469.7
(22) Date de dépôt: 22.10.1993
(51) Int. Cl.: A61F 5/01

(54) **APPAREIL D'ASSISTANCE OU DE SUPPLEANCE DU GENOU**
HILFS- ODER ERGÄNZUNGSORTHESE DES KNIES
KNEE SUPPORT DEVICE OR PROSTHESIS

(30) Priorité: 23.10.1992 BE 9200918
(43) Date de publication de la demande: 09.08.1995
(73) Titulaire: DUMONT, Francoise Ghislaine, B-1030 Bruxelles (BE)
(72) Inventeur: POSTELMANS, Roberto, Jean, José, B-1030 Bruxelles (BE)
(74) Mandataire: Claeys, Pierre
(86) Numéro de dépôt international: BE9300066
(87) Numéro de publication internationale: WO9409729

(56) Documents cités:
- WO-A-92/15264
- FR-A- 2 600 528
- US-A- 3 779 654
- US-A- 5 107 824

## Description

La présente invention est relative à un appareil d'assistance ou de suppléance de genou, comprenant
- au moins une articulation extérieure et une articulation intérieure qui sont disposées de part et d'autre d'un plan vertical antéro-postérieur et qui comportent chacune au moins une pièce proximale et au moins une pièce distale mutuellement articulées,
- des premiers moyens de liaison pour relier mutuellement les pièces proximales des articulations et des deuxièmes moyens de liaison pour relier mutuellement les pièces distales des articulations, les moyens de liaison réalisant un positionnement mutuel des articulations suivant un axe commun,
- des moyens d'attache pour fixer les pièces proximales à une partie fémorale d'un membre inférieur et les pièces distales à une partie tibiale dudit membre inférieur,
- et des moyens de guidage des pièces mutuellement articulées permettant un mouvement relatif de flexion, un roulement antéro-postérieur conjugué à un glissement, une rotation et/ou un mouvement de varus entre lesdites parties fémorale et tibiale, les moyens de guidage fournissant aux pièces de chacune desdites articulations un mouvement différent de celui de chacune des autres articulations,
- lesdits moyens de guidage de chaque articulation comportant au moins une surface de guidage de courbure prédéterminée dans une pièce articulée et au moins un élément suiveur correspondant sur l'autre pièce articulée, ces pièces articulées de chaque articulation présentant en outre des surfaces d'appui de courbure prédéterminée qui coopèrent pendant ledit mouvement relatif entre les parties fémorale et tibiale.

Suivant l'invention, par appareil d'assistance du genou il faut entendre une orthèse de genou et par appareil de suppléance une prothèse ou une endoprothèse de genou.

Dans ce qui suit, il faut entendre par parties fémorale et tibiale d'un membre inférieur, non seulement les parties d'un membre existant, mais aussi des pièces rigides supérieure et/ou inférieure remplaçant ce membre.

Ainsi la pièce proximale peut venir s'adapter sur une pièce rigide supérieure, fixée au fémur, ou elle peut se prolonger le long du fémur et s'y fixer et la pièce distale peut venir s'adapter sur une pièce rigide inférieure, fixée au tibia, ou elle peut se prolonger le long de celui-ci et s'y fixer.

Dans ce qui suit, il faut aussi entendre, par flexion et extension, un mouvement relatif entre les parties fémorale et tibiale d'un membre autour d'un axe sensiblement horizontal traversant le genou approximativement de gauche à droite. Par rotation, il faut entendre un mouvement relatif entre ces parties autour j'un axe vertical. Par mouvement de varus, il faut entendre un mouvement relatif entre ces parties autour d'un axe horizontal antéro-postérieur. Enfin, par roulement et glissement antéro-postérieur, il faut entendre un mouvement relatif entre les extrémités des parties tibiale et fémorale dans un plan antéro-postérieur.

Les orthèses sont des appareils orthopédiques qui permettent d'assurer le mouvement et l'articulation d'un membre déficient d'une personne handicapée. De tels appareils sont constitués de deux parties rigides, reliées entre elles par une articulation au niveau du genou, et ce d'un côté de la jambe ou des deux côtés. Le tout peut être fixé par des bracelets ou des coquilles qui longent la cuisse et la jambe. Les orthèses utilisées pour les membres inférieurs ont couramment deux articulations au niveau du genou, symétriques, qui ne leur permettent qu'un mouvement de flexion et au mieux simultanément un déplacement antéro-postérieur de la partie proximale sur la partie distale (v. EP-A-0297766, US-A-3.779.654). Or, le mouvement physiologique du genou est tridimensionnel, et ces orthèses provoquent inévitablement des tensions sur les ligaments qui vont à l'encontre d'un traitement médical ou chirurgical.

On connaît par ailleurs des orthèses multiaxiales pourvues de plaques planes rainurées coopérantes, permettant successivement un glissement vers l'arrière du tibia par rapport au fémur au début de la flexion, puis une rotation (WO-A-92/15264).

Il existe aussi une genouillère polyaxiale, qui est tridimensionnelle (v. genouillère Poli-Axiale de Generation II Orthotics, Inc., Vancouver, Canada) et qui comporte une articulation à adapter d'un côté du genou déficient. Dans cette articulation, deux segments de sphère, présentant deux rainures en arc de cercle disposées autour d'un axe de manière désaxée, coopèrent. Dans les premiers degrés de flexion, c'est la pièce proximale qui se déplace, et arrivée à la fin de l'amplitude de ses rainures, la flexion continue sur la rainure du deuxième segment de sphère, donnant ainsi un mouvement tridimensionnel, mais non physiologique. En effet, le mouvement physiologique du genou a, grâce au système de bielles des ligaments, un mouvement de roulement antéro-postérieur et de glissement conjugué à une rotation et à un mouvement de varus. La rotation et le mouvement de varus ont lieu, d'après la majorité des études médicales, principalement au début de la flexion, ce que ne permet pas cet appareil connu.

On connaît aussi une orthèse telle que décrite au début (US-A-5107824) où les moyens de guidage sont formés de coques sphériques rainurées coopérant l'une dans l'autre. Etant donné que le centre des coques de l'articulation externe de cet appareil est situé à un autre endroit que le centre des coques de l'articulation interne on en arrive inévitablement à un blocage de l'appareil puisque les coques externes et internes sont aussi reliées l'une à l'autre de manière solidaire par des bracelets rigides autour de la cuisse et de la jambe. De plus la forme sphérique des coques et leur appui intime l'une sur l'autre empêche l'obtention d'un mouvement à trajectoire complexe permettant de suivre le mouvement physiologique du genou.

Les prothèses sont des appareils orthopédiques qui permettent d'assurer la marche à des personnes ayant perdu partiellement ou totalement leur jambe. Ces appareils surmontés d'une emboîture permettent à l'handicapé de s'appuyer sur son moignon et une articulation, située au niveau de l'axe du genou, permet la flexion de la prothèse. Pour les prothèses fémorales pour amputation longue ou pour désarticulation de genou, il est impossible de placer l'articulation prothétique à son emplacement physiologique. L'articulation prothétique est placée plus bas que l'axe physiologique du genou, ce qui rend la marche inconfortable et donne à la position assise une allure inesthétique, la partie de la cuisse étant anormalement plus longue que la partie du tibia.

La présente invention a pour but de mettre au point un appareil d'assistance ou de suppléance de genou perfectionné, qui permette de contrôler le mouvement tridimensionnel avec un roulement antéro-postérieur et un glissement, associé à une rotation et à un varus des parties tibiale et fémorale, le tout parfaitement conjugué lors de la flexion ou de l'extension de la jambe. De plus, l'appareil selon l'invention doit de préférence présenter un très faible encombrement et n'entraîner aucune gêne lors de son fonctionnement.

La présente invention a aussi pour but d'éviter les inconvénients cités ci-dessus.

On a prévu pour cela, suivant l'invention, un appareil d'assistance ou de suppléance de genou, tel que décrit au début, et caractérisé par le fait que
- lesdites surfaces d'appui des moyens de guidage de chaque articulation présentent des courbures complexes mutuellement différentes par lesquelles elles sont en contact uniquement partiel, en au moins trois points, pendant le mouvement entre les parties fémorale et tibiale.

Ainsi qu'on l'a déjà exposé, le mouvement physiologique du genou est composé de plusieurs mouvements simultanés, ce qui est rendu possible par une configuration spéciale des extrémités des os et par une liaison de ceux-ci par des ligaments croisés.

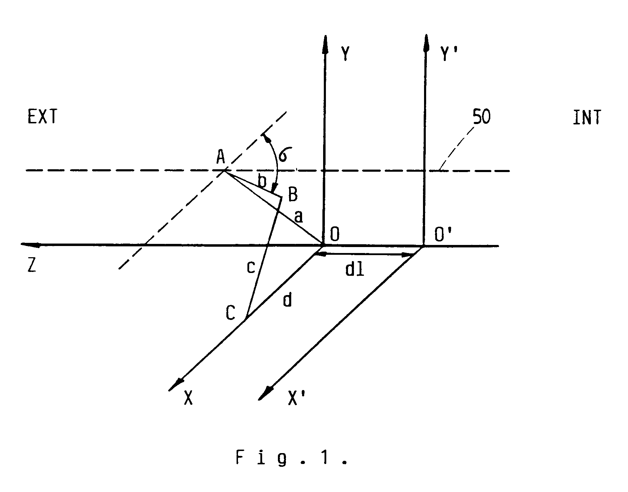

Si l'on se réfère à la figure 1 annexée, on pourra voir l'illustration d'un plan XOY représentant le plan central antéro-postérieur d'un genou gauche, vu de dos. Dans la position représentée, des bielles croisées AO et BC représentent de manière schématique les ligaments croisés susdits en position d'extension de la jambe. La référence a désigne le ligament croisé antérieur et la référence c le ligament croisé postérieur. La référence b (AB) représente le toit intercondylien, et la référence d (CO) le plateau tibial. L'angle σ est l'angle d'inclinaison entre le toit intercondylien et le plan parallèle au plan ZOX, par lequel passe l'axe 50, qui lui-même passe en A.

Selon certaines études théoriques, une rotation du genou s'effectue, simultanément à la flexion, autour d'un axe sensiblement vertical (O'Y') situé dans un plan, appelé plan pivot, lui-même disposé légèrement vers l'intérieur par rapport au plan central. Le varus s'effectue autour de l'axe O'X' situé dans ce même plan pivot. Sur la figure 1, le genou représenté est un genou gauche, l'intérieur du genou se trouvant à droite sur le dessin et le plan pivot Y'O'X' étant donc décalé d'une petite distance dl par rapport au plan central YOX.

L'application de deux embiellages identiques de part et d'autre du genou, dans le même axe du genou, ne peut donner lieu qu'à une flexion autour de l'axe OZ, sans rotation ni varus. L'agencement des bielles permet éventuellement en plus un roulement antéro-postérieur et un glissement. L'invention a consisté à mettre au point un appareil d'assistance ou de suppléance du genou comportant des moyens de guidage qui fournissent aux pièces articulées un mouvement du type de celui obtenu par deux embiellages différents de part et d'autre d'un plan antéro-postérieur.

Pour illustrer cet agencement on pourrait dire que l'on a mis sur une voiture, sur un même axe, deux roues avant dont la droite par exemple est plus grande que la gauche. Cette voiture va nécessairement tourner vers la gauche, autour d'un centre unique pour toute la voiture, d'une manière contrôlée par le diamètre de chaque roue. On peut également envisager, le long de cet axe, une infinité de roues qui auront chacune un rayon différent suivant leur emplacement, pour respecter le mouvement général.

Ainsi qu'on le comprendra aisément de la figure 1, dans chaque plan parallèle au plan central antéropostérieur s'écartant vers Z l'embiellage doit permettre un mouvement d'un rayon plus grand pour autoriser simultanément la rotation et le varus autour de leur axe correspondant situé dans le plan pivot et il fournit un mouvement de rayon plus petit dans chaque plan parallèle au plan central allant dans le sens opposé.

Enfin, il faut que ce mouvement de type embiellage s'inscrive dans des surfaces courbes complexes pour permettre sans problème les mouvements simultanés de rotation et de varus. Il faut en effet tenir compte, comme on l'a déjà dit, que ces mouvements de rotation et de varus, s'ils sont simultanés principalement au début de la flexion, varient d'amplitude au fur et à mesure de la flexion, et chacun de manière différente dans le temps.

Il est apparu que l'application de simples plaques planes rainurées ou de calottes sphériques rainurées, articulées l'une dans l'autre et en appui l'une sur l'autre par toute leur surface, ne permettait pas d'accompagner ce mouvement irrégulier et que nécessairement à un moment ou l'autre l'appareil orthétique devait se bloquer ou le genou devait être forcé par un guidage inadapté des surfaces planes ou sphériques coopérantes.

Suivant une forme perfectionnée de réalisation de l'invention les pièces articulées d'une articulation comportent chacune, à une première extrémité, au moins une coque munie d'au moins une rainure et/ou d'au moins un élément suiveur capable de coulisser dans une rainure de l'autre pièce articulée, et au moins une coque d'une des pièces articulées comporte une surface d'appui présentant une première courbure complexe prédéterminée avec laquelle une surface d'appui présentant une deuxième courbure complexe, différente de la première, d'au moins une coque de l'autre pièce articulée peut coopérer par ledit contact partiel pendant ledit mouvement relatif entre les parties fémorale et tibiale. Pour permettre la rotation et le varus du genou, dans le cas de l'utilisation suivant l'invention d'embiellages différents de part et d'autre du genou, il faut, pour éviter un blocage des embiellages, fournir à ceux-ci un certain jeu. Malheureusement cela ne peut se faire qu'avec une certaine perte de contrôle du mouvement du genou. C'est la raison pour laquelle, suivant une forme préférentielle de l'invention, on applique des mécanismes fournissant aux pièces articulées un mouvement du type obtenu par un embiellage, tout en étant parfaitement contrôlé dans l'espace. Il s'agit par exemple de cames, de préférence des cames rainurées, et d'éléments suiveurs de came, de sangles croisées mises sous tension ou de toute articulation analogue. Les rainures ou surfaces de guidage sont pratiquées dans des cames tridimensionnelles. Les cames rainurées ont ainsi de préférence une forme de coque de courbure complexe prédéterminée en fonction du mouvement à obtenir.

Il faut noter à ce propos que les spécialistes ne sont pas toujours d'accord sur les mouvements qui se produisent au cours de la flexion d'un genou ou en tout cas sur leur amplitude au moment où ils se produisent. Par ailleurs chaque patient présente des genoux de paramètres différents. La forme et la courbure des rainures et des coques seront à calculer en fonction de cela, ainsi que leur zone de contact partiel pendant leur coopération.

Suivant une forme de réalisation de l'invention, le plan vertical antéro-postérieur susdit traverse le genou. Dans le cas d'une orthèse, l'articulation extérieure se trouvera avantageusement du côté extérieur du genou et l'articulation intérieure du côté intérieur du genou. Les articulations seront donc ici de part et d'autre du plan pivot.

Suivant une autre forme de réalisation de l'invention, le plan vertical antéro-postérieur susdit est disposé d'un côté du genou. On peut en effet imaginer que l'articulation extérieure et l'articulation intérieure puissent se trouver toutes deux d'un même côté du genou.

Suivant encore une autre forme de réalisation de l'invention, les pièces proximales des articulations extérieure et intérieure forment un élément d'une pièce, les pièces distales de ces articulations forment aussi un élément d'une pièce et les deux éléments d'une pièce sont articulés l'un sur l'autre par lesdits moyens de guidage.

D'autres formes de réalisation sont indiquées dans les revendications secondaires.

D'autres détails et particularités de l'invention ressortiront de la description donnée ci-après à titre non limitatif et avec référence aux dessins annexés.

La figure 1 représente de manière schématique un embiellage croisé d'une articulation de genou gauche.

Les figures 2 à 5 représentent en élévation et en profil les pièces articulées d'une articulation intérieure d'une orthèse gauche suivant l'invention.

Les figures 6 à 9 représentent en élévation et en profil les pièces articulées de l'articulation extérieure de cette orthèse.

Les figures 10 et 11 représentent une vue en élévation, à partir du plan médian, des deux articulations à l'état monté, l'une en position d'extension et l'autre après une flexion d'environ 40°.

Les figures 12 et 13 représentent une vue de devant de l'orthèse, en position d'extension et en position de flexion d'environ 40°.

La figure 14 représente une vue du dessus de l'orthèse en position de flexion d'environ 40°.

La figure 15 représente une vue d'une variante de réalisation d'articulation d'une orthèse suivant l'invention.

Les figures 16 à 18 représentent une variante de réalisation d'appareil suivant l'invention adapté pour une prothèse.

Les figures 19 à 21 sont des graphiques destinés à illustrer le mouvement dans l'espace de l'axe 50 représenté sur la figure 1.

La figure 22 représente un graphique semblable à la figure 19, illustrant diverses variantes de réalisation de l'invention.

La figure 23 représente dans un graphique les mouvements relatifs de deux points l'un situé sur une coque d'une articulation, l'autre sur l'autre coque.

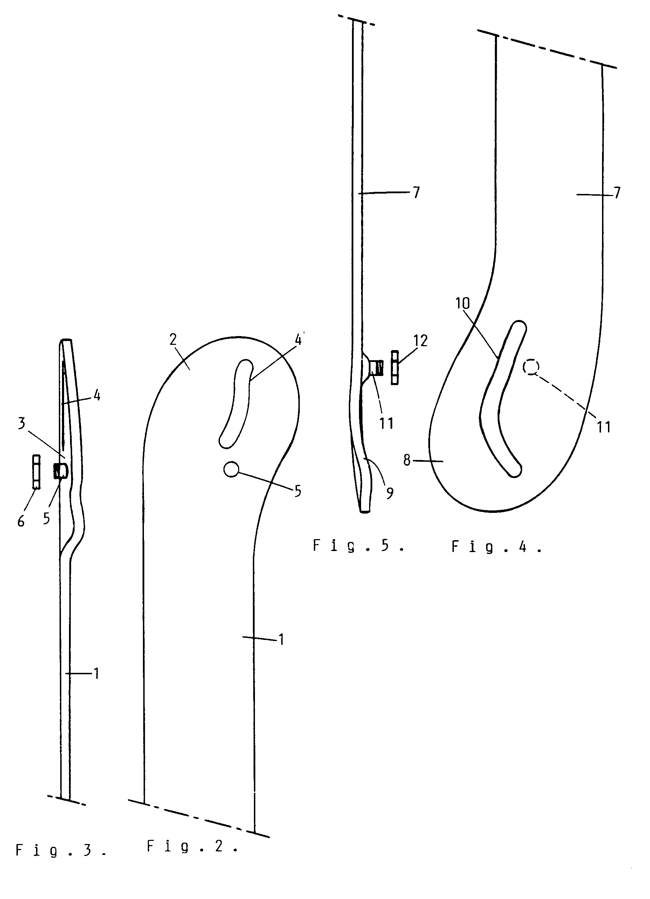

Les figures 2 et 3 représentent des vues en élévation et de profil de la pièce distale de l'articulation intérieure de cette orthèse. Cette pièce distale est formée d'une tige 1 dont une extrémité, l'extrémité proximale, s'évase en forme d'une coque 2 présentant une surface extérieure courbe 3 de courbure prédéterminée. Cette coque 2 est munie d'une rainure de guidage 4 présentant une courbure prédéterminée et d'un goujon 5 faisant saillie latéralement par rapport à la surface courbe 3. Ce goujon est, dans cet exemple de réalisation, fileté et un écrou 6 peut être vissé dessus.

Les figures 4 et 5 représentent des vues en élévation et en profil de la pièce proximale de l'articulation intérieure de cette orthèse. Cette pièce proximale est formée d'une tige 7 dont une extrémité, l'extrémité distale, s'évase en forme d'une coque 8 présentant une surface intérieure courbe 9 de courbure prédéterminée. Cette coque 8 est munie d'une rainure de guidage 10 présentant une courbure prédéterminée et d'un goujon 11 faisant saillie latéralement par rapport à la surface courbe 9. Ce goujon est, dans cet exemple de réalisation, fileté et un écrou 12 peut être vissé dessus.

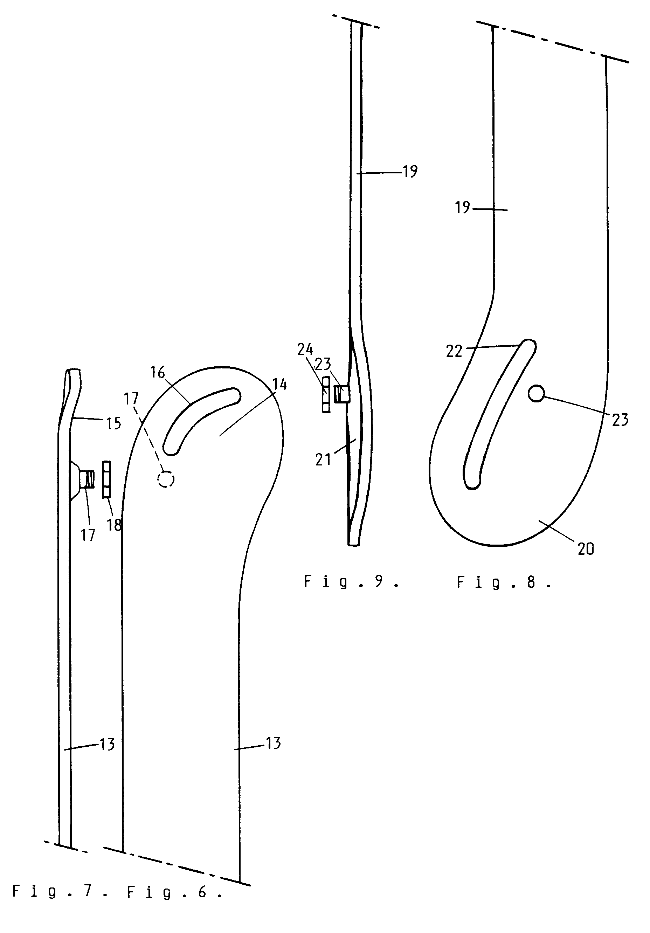

Les figures 6 et 7 représentent des vues en élévation et en profil de la pièce distale de l'articulation extérieure de cette orthèse. Cette pièce distale est formée d'une tige 13 dont une extrémité, l'extrémité proximale, s'évase en forme d'une coque 14 présentant une surface extérieure courbe 15 de courbure prédéterminée. Cette coque 14 est munie d'une rainure de guidage 16 présentant une courbure prédéterminée et d'un goujon 17 faisant saillie latéralement par rapport à la surface courbe 15. Ce goujon 17 est ici aussi fileté et un écrou 18 peut être vissé dessus.

Les figures 8 et 9 représentent des vues en élévation et en profil de la pièce proximale de l'articulation extérieure de cette orthèse. Cette pièce proximale est formée d'une tige 19 dont une extrémité, l'extrémité distale, s'évase en forme d'une coque 20 présentant une surface extérieure courbe 21 de courbure prédéterminée. Cette coque 20 est munie d'une rainure de guidage 22 présentant une courbure prédéterminée et d'un goujon 23 faisant saillie latéralement par rapport à la surface courbe 21. Ce goujon 23 est ici aussi fileté et un écrou 24 peut être vissé dessus.

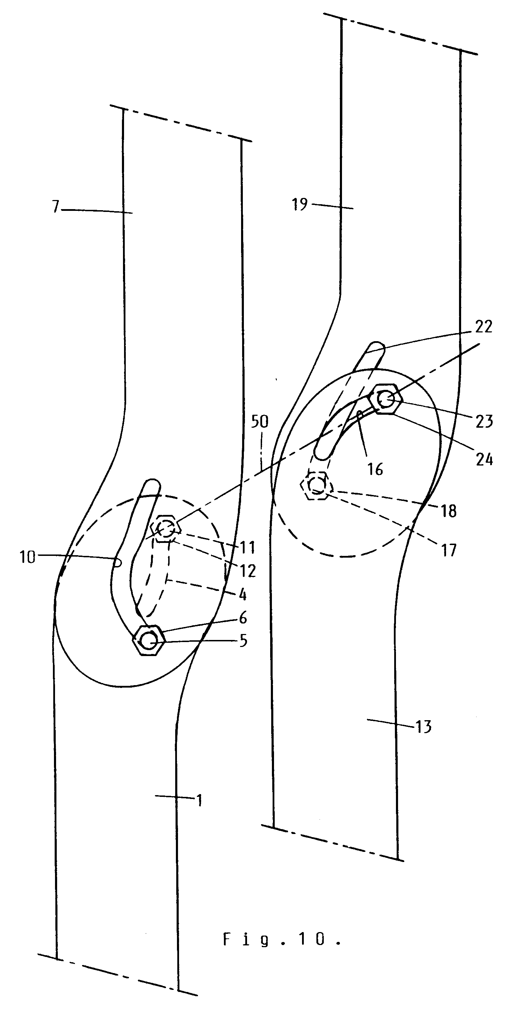

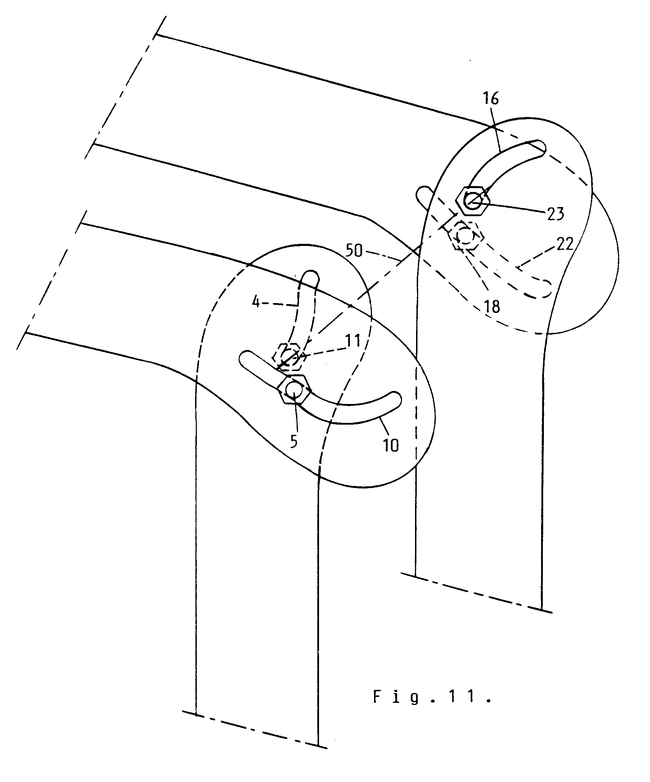

Les figures 10 et 11 sont des vues en fausse perspective des articulations à l'état assemblé. Les deux articulations sont représentées dans ces deux figures suivant une vue en élévation à partir du plan médian du corps et elles sont représentées l'une à côté de l'autre, l'articulation intérieure à gauche et l'articulation extérieure à droite.

Il faut noter que, dans toutes les figures représentant un état assemblé de l'appareil d'assistance ou de suppléance du genou suivant l'invention, on représente pour des raisons de facilité uniquement la pièce distale en position droite, invariable, et la pièce proximale se déplace par rapport à elle.

A l'état assemblé, et dans la position d'extension de l'appareil, dans l'articulation intérieure le goujon 5 se trouve dans la rainure 10, à une extrémité de celle-ci, et il traverse la coque 8, tandis que le goujon 11 pénètre dans la rainure 4 à une extrémité de celle-ci et traverse la coque 2. Les deux coques sont maintenues l'une en face de l'autre par les écrous 6 et 12 qui sont vissés sur les extrémités en saillie des goujons 5 et 11. De même, dans l'articulation extérieure, le goujon 17 se trouve dans la rainure 22, à une extrémité de celle-ci, et il traverse la coque 20 tandis que le goujon 23 se trouve dans la rainure 16, à une extrémité de celle-ci, et traverse la coque 14.

Les deux articulations sur la figure 11 sont représentées à l'état assemblé également, mais après une flexion d'environ 20°. Dans le cas illustré, le goujon 11 se trouve alors à proximité de l'autre extrémité de la rainure 4 et le goujon 5 approximativement aux 2/3 de la trajectoire de la rainure 10. De même, le goujon 23 se trouve à proximité de l'autre extrémité de la rainure 16 et le goujon 18 approximativement aux 2/3 de la trajectoire de la rainure 22. Comme on peut le remarquer, les parties des rainures 10 et 22, qui n'ont pas encore été parcourues par les goujons 5 et 18, sont sensiblement rectilignes. Cela signifie que, dans le présent exemple de réalisation, les articulations vont, à partir d'un certain moment, suivre uniquement un mouvement de flexion avec un roulement antéro-postérieur et un glissement. En fait, dans cet exemple de réalisation, les rainures de guidage des coques pendant les deux premiers tiers de leur trajectoire et l'emplacement des goujons servant d'éléments suiveurs ont été calculés en fonction du mouvement à obtenir, mouvement qui est du type de celui que l'on obtiendrait par un embiellage à deux bielles. Les mouvements obtenus par ces moyens de guidage des pièces articulées sont différents pour l'articulation intérieure et pour l'articulation extérieure. Il en résulte que, pendant les 20 premiers degrés de la flexion, simultanément, une rotation et un varus sont imposés par l'appareil, dans l'exemple de réalisation illustré.

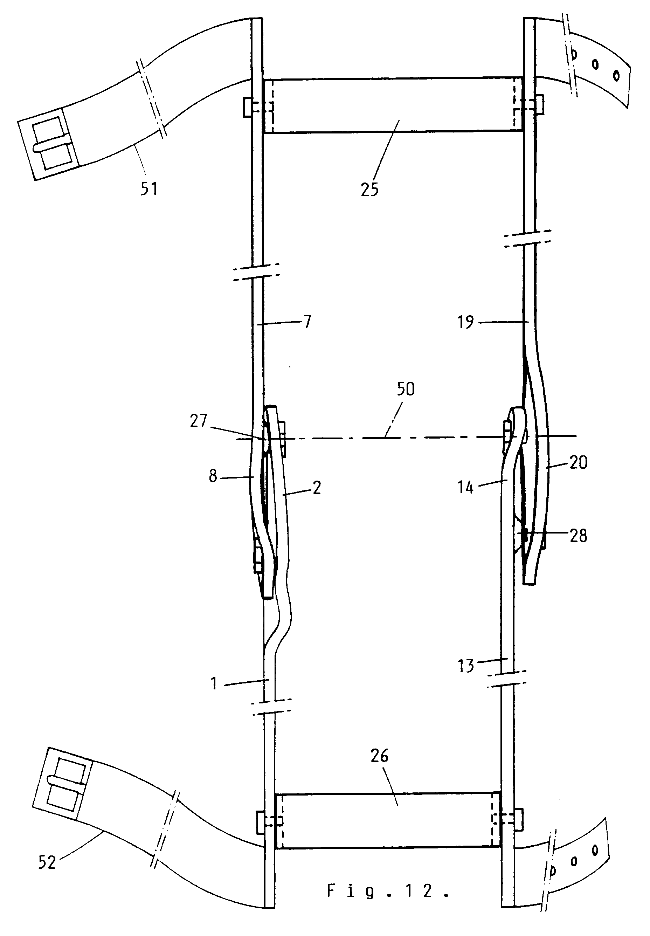

Ainsi qu'il ressort de la figure 12, les extremités des pièces proximales et distales des deux articulations de l'orthèse, qui sont opposées aux coques, sont mutuellement reliées par des moyens de liaison. Ceux-ci permettent la réalisation d'un positionnement mutuel des articulations, de façon que celles-ci soient disposées suivant un axe commun. Dans l'exemple de réalisation illustré, ces moyens de liaison consistent en des bracelets rigides 25 et 26. Dans cette position l'axe commun des articulations de cet exemple de réalisation est représenté en traits interrompus et désigné par la référence 50 (voir aussi figures 1, 10 et 11, d'où il ressort que cet axe se déplace lui aussi au cours de la flexion ou de l'extension).

Il est bien entendu que ces moyens de liaison pourraient se présenter sous diverses formes. Par exemple, on pourrait prévoir une forme de réalisation dans laquelle les moyens de liaison et les pièces proximales ou respectivement distales sont réalisées d'une pièce.

D'une manière connue, des moyens d'attache, dans l'exemple illustré ici, des sangles 51 et 52 permettent la fixation de l'appareil de suppléance du genou à la cuisse et à la jambe du patient.

Les courbures des coques de chaque articulation sont différentes, et elles ne peuvent être en contact mutuel par la totalité de leurs surfaces qui se font face. A tout moment du mouvement de flexion et d'extension, les deux coques sont en appui mutuel en au moins trois endroits. Si l'on prend par exemple l'articulation intérieure de l'orthèse illustrée, les surfaces d'appui des deux coques 2 et 8 sont situées à proximité du goujon 5 là où il est situé dans la rainure 10, à proximité du goujon 11 là où il est situé dans la rainure 4 et, par exemple en position d'extension (voir figure 12), à l'extrémité inférieure de la coque 8 qui est en contact avec un renflement latéral de la coque 2. Etant donné qu'à ces endroits le frottement est plus intense, il faut veiller avantageusement à ce qu'au moins ces surfaces partielles d'appui entre les coques présentent une résistance à l'usure efficace. On peut notamment prévoir par endroits une surépaisseur dans une des coques ou dans les deux, comme les surépaisseurs 27 et 28 dans les coques 8 et 14 respectivement.

Ainsi qu'il ressort de la figure 12, dans la position d'extension, les pièces proximales 7 et 19 sont, dans cet exemple, parallèles aux pièces distales 1 et 13.

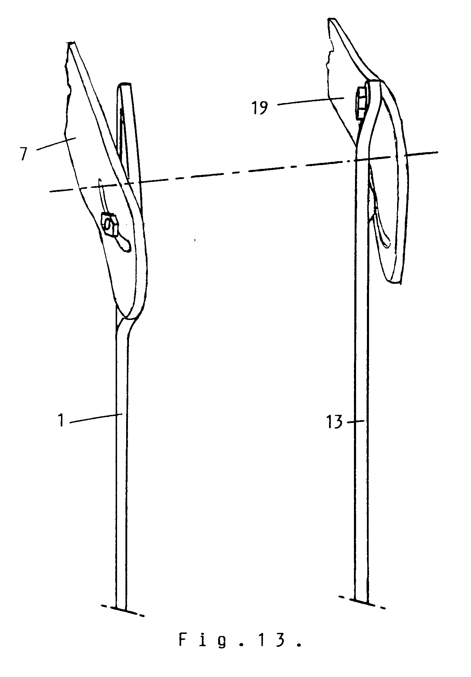

Sur la figure 13, qui représente, comme la figure 12, une vue de face de l'appareil suivant l'invention, mais après une flexion d'environ 40°, les pièces proximales 7 et 19 ont effectué, en plus de la flexion, du roulement antéro-postérieur et du glissement et de la rotation, un mouvement de varus. En effet, il ressort de cette figure, que les pièces proximales 7 et 19 ont basculé vers le bas et vers la gauche sur le dessin autour d'un axe antéro-postérieur, non représenté, et qui est perpendiculaire au dessin.

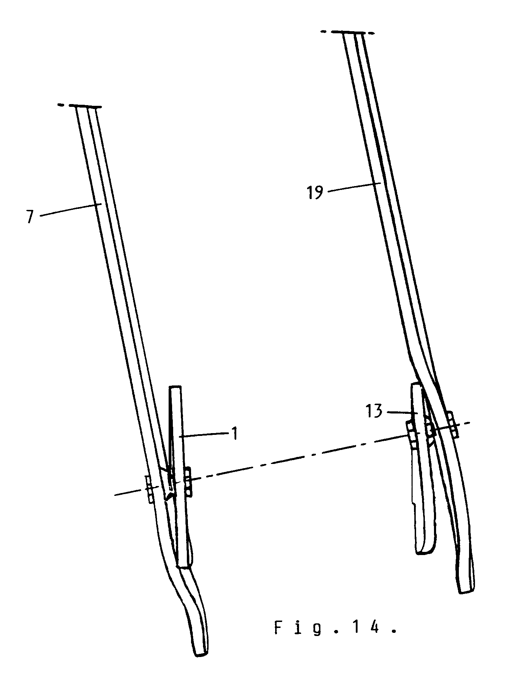

Sur la figure 14, qui représente une vue du dessus de l'appareil suivant l'invention, après une flexion d'environ 40°, les pièces proximales 7 et 19 ont effectué, simultanément à la flexion, au roulement antéropostérieur et au glissement ainsi qu'au varus, un mouvement de rotation. En effet, il ressort de cette figure que les pièces proximales 7 et 19 ont tourné vers la gauche sur le dessin autour d'un axe vertical, non représenté, et qui est perpendiculaire au dessin.

Le contact uniquement partiel nécessaire entre les coques pendant leur coopération mutuelle ressort particulièrement bien de cette figure 14.

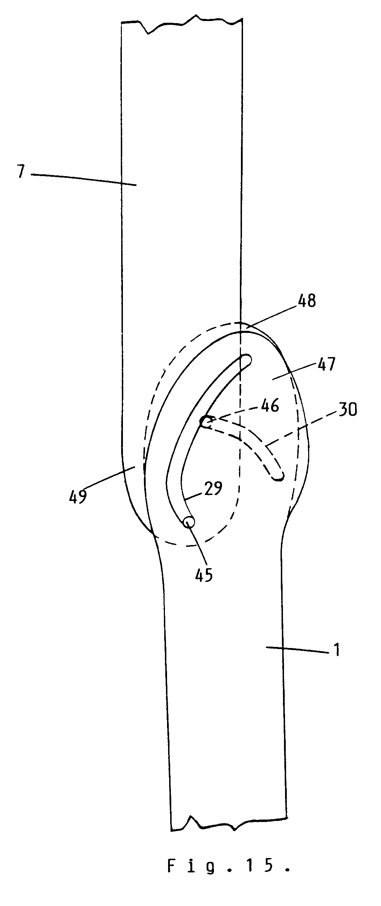

La figure 15 illustre une variante de réalisation d'une articulation d'un appareil suivant l'invention. Dans cet exemple de réalisation, la tige 1 de la pièce distale se termine à son extrémité proximale en forme de deux coques 47 et 48 présentant des surfaces qui se font face, à courbure prédéterminée. La tige 7 de la pièce proximale se termine en une seule coque 49 qui est intercalée entre les coques 47 et 48. La coque 49 présente deux surfaces de courbure prédéterminée capables de coopérer avec l'une desdites surfaces se faisant face des deux coques 47 et 48. Dans l'exemple de réalisation illustré, les moyens de guidage sont constitués d'une première rainure 29 à courbure prédéterminée dans la coque 47, d'une deuxième rainure 30 à courbure prédéterminée dans la coque 48 et de deux goujons suiveurs 45, 46 faisant chacun saillie d'une desdites surfaces capables de coopérer de la coque 49 et pénétrant l'un dans la rainure 29 et l'autre dans la rainure 30.

Il est évident que' l'agencement des moyens de guidage pourrait être différent, par exemple les rainures pourraient être sur la coque 49 et les goujons sur les coques 47 et 48. Il faut aussi noter que les courbures des rainures et des coques doivent être calculées en fonction de leur position spatiale par rapport au plan antéro-postérieur appelé le plan pivot. La distance entre ce plan pivot et chacune des coques 47 et 48 est différente et, pour obtenir un mouvement parfaitement contrôlé de l'articulation selon cet exemple de réalisation, il est souhaitable de tenir compte de ce fait lors du calcul des courbures de chacune des coques 47 et 48 et de chacune des rainures de ces coques.

Dans l'exemple de réalisation illustré sur les figures 2 à 14, le genou handicapé est destiné à être logé entre les articulations extérieure et intérieure qui sont ainsi disposées de part et d'autre d'un plan vertical antéro-postérieur divisant le genou. On peut toutefois imaginer que ce plan vertical antéro-postérieur soit disposé en dehors du genou, latéralement par rapport à celui-ci. Les deux articulations se trouvent alors d'un seul et même côté du plan pivot, mais l'une d'elles est située à plus grande distance que l'autre de ce plan. Cela implique que les moyens de guidage des pièces d'une des deux articulations fournissent à celle-ci un mouvement du type obtenu par un embiellage qui soit différent de celui fourni par les moyens de guidage des pièces de l'autre articulation, si l'on veut obtenir un mouvement parfaitement contrôlé de l'orthèse.

On pourrait par exemple considérer que la forme de réalisation illustrée sur la figure 15 est en soi un appareil suivant l'invention applicable par exemple comme orthèse, d'un seul côté du genou. En effet, on peut considérer qu'ici le plan antéro-postérieur passe au centre de la tige 7, les deux articulations différentes se trouvant de part et d'autre de ce plan. Dans cet exemple, les pièces proximales des deux articulations forment ensemble un élément d'une pièce, c'est-à-dire la tige 7 et la coque 49.

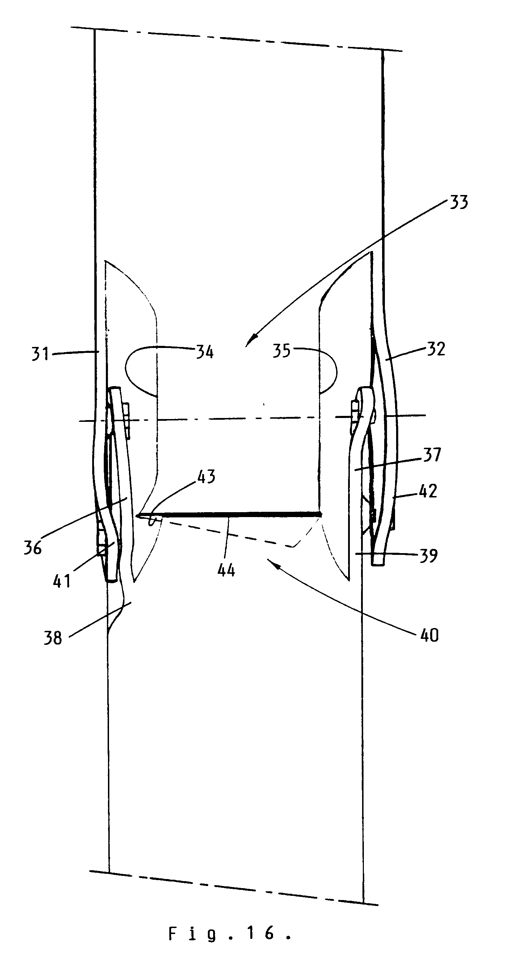

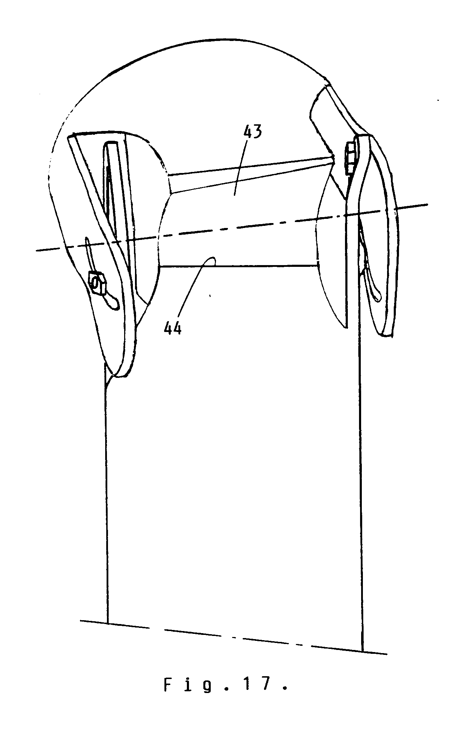

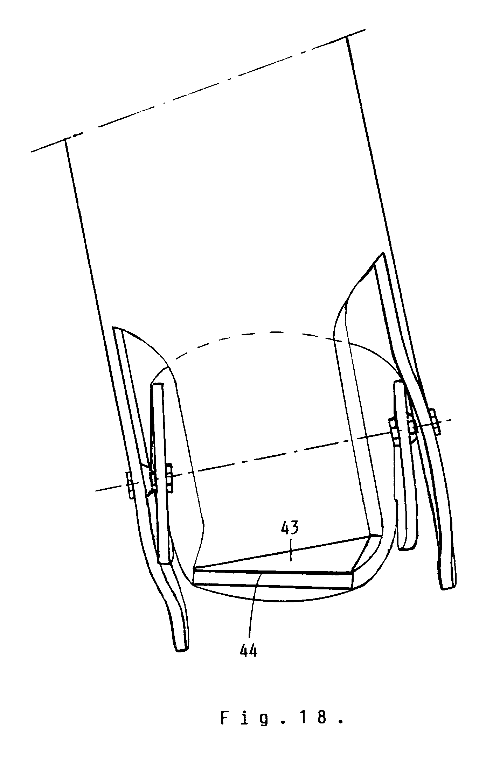

Sur les figures 16 à 18 on a représenté un appareil de prothèse suivant l'invention dans une vue de face en état d'extension et dans une vue de face et une vue du haut en état de flexion, à un angle d'environ 40°.

Dans cet exemple de réalisation, les pièces proximales 31 et 32 de l'articulation intérieure et de l'articulation extérieure forment ensemble un élément d'une pièce ayant l'allure d'un bloc 33. Ce bloc 33 présente deux évidements latéraux 34 et 35 pour le passage des coques 36 et 37 des pièces distales 38 et 39 des articulations intérieure et extérieure. Ces pièces distales 38 et 39 forment elles aussi, dans cet exemple de réalisation, un élément d'une pièce ayant l'allure d'un bloc 40.

Les coques 41 et 42 des pièces proximales et les coques 36 et 37 des pièces distales sont identiques à celles de l'exemple de réalisation d'orthèse représenté sur les figures 2 à 14.

Dans cet exemple de réalisation, les deux blocs 33 et 40 sont en contact par des surfaces de guidage 43 et 44 capables de glisser l'une sur l'autre pendant le mouvement des articulations.

On pourrait aussi prévoir dans l'un des blocs, par exemple le bloc 33, une rainure de guidage le traversant de gauche à droite en présentant une surface de guidage correspondant à la surface de guidage 41. La jambe artificielle pourrait porter deux coques à disposer de part et d'autre du bloc 33 et à réunir par un axe suiveur capable de coulisser dans la rainure de guidage, deux goujons du bloc faisant saillie dans deux rainures prévues dans lesdites coques. Les articulations ne comporteraient alors pas de coques sur les pièces proximales, les parties intérieure et extérieure du bloc ayant la forme des coques, à cet endroit.

On pourrait aussi prévoir des formes de réalisation comportant un nombre d'articulations supérieur à deux, les moyens de guidage de chaque articulation fournissant un mouvement différent en fonction de leur emplacement par rapport au plan pivot.

Pour illustrer l'invention, deux exemples de réalisation non limitatifs de modèles de mouvements à obtenir dans les appareils d'assistance et de suppléance suivant l'invention sont donnés ci-dessous.

### Exemple 1

| Paramètres à respecter : | |
|---|---|
| Longueur du ligament croisé antérieur a | 49 mm |
| Longueur du plateau fémoral b | 20 mm |
| Longueur du ligament croisé postérieur c | 40 mm |
| Longueur du plateau tibial d | 50 mm |
| Angle σ | 33° |
| 1/2 largeur du genou | 55 mm |
| Situation du plan pivot par rapport au plan central, vers le plan médian | 30 % de la 1/2 largeur du genou |

### Evolution des mouvements en fonction de la flexion

| Flexion | Rotation | Varus |
|---|---|---|
| 10° | 3° | 2° |
| 25° | 8° | 5° |
| 45° | 11° | |
| 90° | | 7° |

### Exemple 2

Mêmes paramètres à respecter que le modèle de l'Exemple 1, mais avec une autre évolution des mouvements en fonction de la flexion.

| Flexion | Rotation | Varus |
|---|---|---|
| 10° | 6° | 2° |
| 25° | 11° | 5° |
| 45° | 15° | |
| 90° | | 7° |

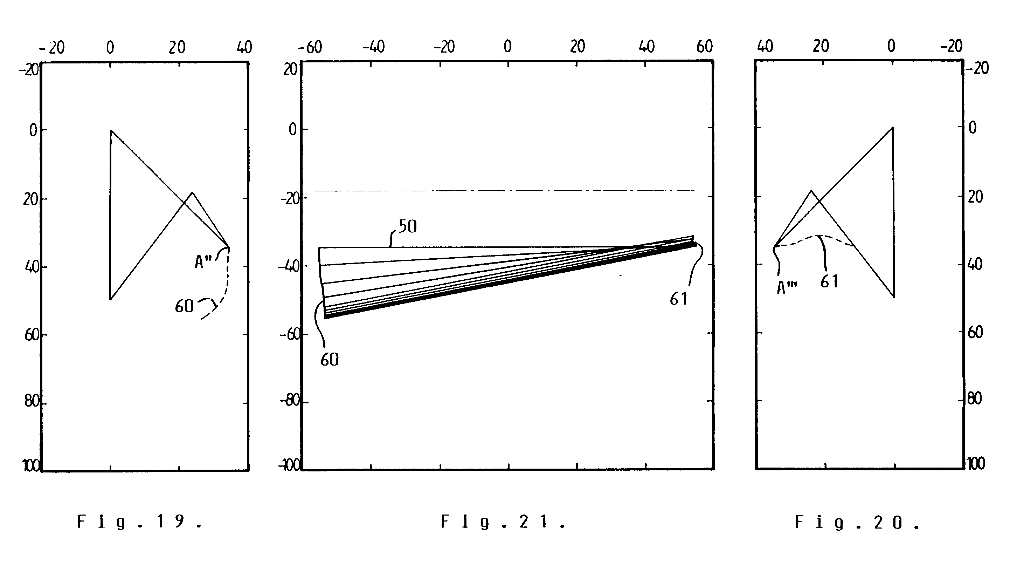

La figure 19 représente un graphique dans un plan parallèle au plan XOY, situé à l'extérieur du genou, où est illustré l'embiellage de la figure 1. Pour permettre sa comparaison avec la figure 21 qui est une vue du dessus d'un plan parallèle au plan XOZ, on a rabattu le graphique de la figure 19 de 90°.

Le trait interrompu désigné par la référence 60 représente une vue latérale du mouvement suivi dans le plan susdit par la point A'', situé sur l'axe 50 en position d'extension (voir figure 1), au cours de la flexion du modèle selon l'Exemple 1. On peut envisager de rainurer par exemple la coque de la pièce distale de l'articulation extérieure de cette manière.

La figure 20 représente un graphique semblable, mais dans un plan parallèle au plan XOY, situé à l'intérieur du genou. Le trait interrompu désigné par la référence 61 représente une vue latérale du mouvement suivi dans ce plan par le point A''', situé sur l'axe 50 en position d'extension (voir figure 1), au cours de la flexion. De même on peut envisager de rainurer par exemple la coque de la pièce distale de l'articulation intérieure de cette manière.

La figure 21 représente une vue du dessus de la partie fixe (tibiale) de l'articulation. En abscisse le point 0 représente la plan central du genou et à proximité du chiffre 20 se trouve le plan pivot. L'axe 50, dont les extrémités sont formées dans l'exemple envisagé de goujons pénétrant dans les rainures 60 et 61, effectue au cours de la flexion un mouvement rendu par la figure 21, où la succession des lignes droites représentent la succession des emplacements de l'axe 50.

Ainsi qu'il ressort de la figure 21, les rainures 60 et 61 ont non seulement une courbure complexe quand elles sont vue latéralement dans un plan, comme sur les figures 19 et 20, mais elles doivent en outre être inscrites dans des surfaces non pas planes ou sphériques, mais dans des surfaces de courbures complexes.

La détermination de ces circonvolutions asymétriques se fait par calcul en fonction des paramètres à respecter.

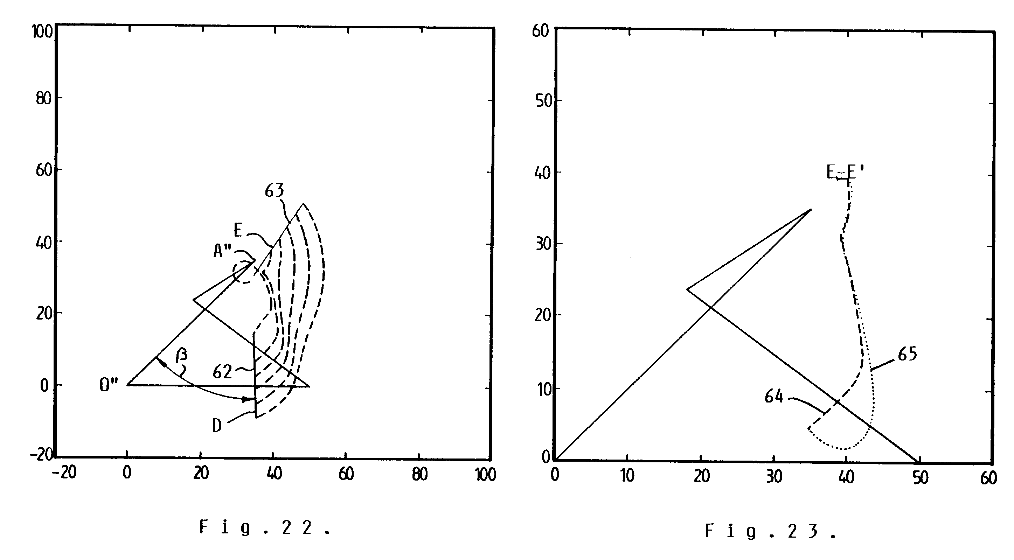

La figure 22 est semblable à la figure 19. Toutefois, ici, le point de départ (position d'extension) sélectionné pour la rainure de la coque de la pièce distale par exemple de la coque extérieure n'est plus le point A'', mais un point D situé sur une droite 62 orientée sous angle β par rapport à la droite O''A''. Selon la situation, choisie sur cette droite, du point de départ les rainures auront des formes différentes que sont illustrées en traits interrompus. L'extrémité E de toutes ces rainures possibles se situe aussi sur le graphique sur une droite 63, mais les courbes obtenues ne sont pas parallèles. On comprendra aisément de ce graphique que les solutions possibles pour le façonnage des différentes articulations est infiniment grand.

La figure 23 est semblable à la figure 22, à plus grande échelle. Elle illustre par le trait interrompu 64 la rainure sélectionnée, son point de départ D et son extrémité opposée E. Sur l'autre coque, celle de la pièce proximale de l'articulation, en position d'extension un point E' existe en face de l'extrémité de la rainure 64 de la pièce distale. Pendant le mouvement de flexion, ce point E' va lui aussi subir un mouvement qui est illustré par le trait pointillé 65. Il en ressort que le point E' doit pouvoir suivre une autre trajectoire que celle de la rainure 64, ce qui implique des formes de coques permettant un contact uniquement partiel entre les surfaces d'appui de celles-ci.

## Revendications

1. Appareil d'assistance ou de suppléance de genou, comprenant
- au moins une articulation extérieure et une articulation intérieure qui sont disposées de part et d'autre d'un plan vertical antéro-postérieur et qui comportent chacune au moins une pièce proximale (7,8; 19,20; 49; 31-33) et au moins une pièce distale (1,2; 13,14; 47,48; 38-40) mutuellement articulées,
- des premiers moyens de liaison (25) pour relier mutuellement les pièces proximales des articulations et des deuxièmes moyens de liaison (26) pour relier mutuellement les pièces distales des articulations, les moyens de liaison (25,26) réalisant un positionnement mutuel des articulations suivant un axe commun (50),
- des moyens d'attache (51,52) pour fixer les pièces proximales à une partie fémorale d'un membre inférieur et les pièces distales à une partie tibiale dudit membre inférieur,
- et des moyens de guidage des pièces mutuellement articulées permettant un mouvement relatif de flexion, un roulement antéro-postérieur conjugué à un glissement, une rotation et/ou un mouvement de varus entre lesdites parties fémorale et tibiale, les moyens de guidage fournissant aux pièces de chacune desdites articulations un mouvement différent de celui de chacune des autres articulations,
- lesdits moyens de guidage de chaque articulation comportant au moins une surface de guidage (4,10,16,22; 29,30) de courbure prédéterminée dans une pièce articulée et au moins un élément suiveur (5,11,17,23; 45,46) correspondant sur l'autre pièce articulée, ces pièces articulées de chaque articulation présentant en outre des surfaces d'appui (3,9,15,21) de courbure prédéterminée qui coopèrent pendant ledit mouvement relatif entre les parties fémorale et tibiale,
caractérisé en ce que lesdites surfaces d'appui des moyens de guidage de chaque articulation présentent des courbures complexes mutuellement différentes par lesquelles elles sont en contact uniquement partiel, en au moins trois points, pendant le mouvement entre les parties fémorale et tibiale.

2. Appareil suivant la revendication 1, caractérisé en ce que l'une des pièces articulées (1, 47, 48) d'une des articulations présente deux surfaces de guidage (29, 30) de courbures prédéterminées et en ce qu'une autre des pièces articulées (7, 49) de cette articulation présente deux éléments suiveurs (45, 46) capables de suivre chacun l'une desdites deux surfaces de guidage (29, 30).

3. Appareil suivant l'une ou l'autre des revendications 1 et 2, caractérisé en ce que les pièces articulées d'une articulation comportent chacune, à une extrémité, au moins une coque (2, 8, 14, 20, 47-49, 36, 37, 41, 42) munie d'au moins une rainure (4, 10, 16, 22, 29, 30) et/ou d'au moins un élément suiveur (5, 11, 17, 23, 45, 46) capable de coulisser dans une rainure de l'autre pièce articulée, et en ce qu'au moins une coque (2, 14, 47-48, 36, 37) d'une des pièces articulées comporte une surface d'appui (3, 15) présentant une première courbure complexe prédéterminée avec laquelle une surface d'appui (9, 21) présentant une deuxième courbure complexe, différente de la première, d'au moins une coque (8, 20, 49, 41-42) de l'autre pièce articulée peut coopérer par ledit contact partiel pendant ledit mouvement relatif entre les parties fémorale et tibiale.

4. Appareil suivant la revendication 3, caractérisé en ce que l'une des pièces articulées d'une articulation comporte deux coques (47, 48) qui sont disposées l'une en face de l'autre et munies chacune d'une rainure (29, 30) et qui comportent des surfaces d'appui se faisant face à courbure prédéterminée et en ce que l'autre des pièces articulées de cette articulation comporte une coque (49) qui est intercalée entre les deux coques (47, 48) susdites et est munie de deux éléments suiveurs (45, 46) pénétrant chacun dans une desdites rainures (29, 30), et qui présente deux surfaces d'appui de courbure différente des courbures des surfaces d'appui se faisant face, ces surfaces d'appui étant chacune capables de coopérer par un contact partiel susdit avec l'une desdites surfaces d'appui se faisant face des deux coques pendant ledit mouvement relatif entre les parties fémorale et tibiale.

5. Appareil suivant l'une quelconque des revendications 1 à 4, caractérisé en ce que les éléments suiveurs (5, 6, 11, 12, 17, 18, 23, 24) servent simultanément de moyens de retenue des pièces articulées articulées en contact partiel l'une contre l'autre.

6. Appareil suivant l'une des revendications 1 à 5, caractérisé en ce que le plan vertical antéro-postérieur susdit traverse le genou.

7. Appareil suivant l'une des revendications 1 à 4, caractérisé en ce que le plan vertical antéro-postérieur susdit est disposé d'un côté du genou.

8. Appareil suivant l'une des revendications 1, 2 et 5, caractérisé en ce que les pièces proximales des articulations susdites forment un élément d'une pièce et en ce que les pièces distales de ces articulations sont articulées sur l'élément d'une pièce par lesdits moyens de guidage.

9. Appareil suivant l'une des revendications 1, 2 et 5, caractérisé en ce que les pièces distales des articulations susdites forment un élément d'une pièce et en ce que les pièces proximales de ces articulations sont articulées sur l'élément d'une pièce par lesdits moyens de guidage.

10. Appareil suivant l'une des revendications 1, 2 et 5, caractérisé en ce que les pièces proximales (31-33) des articulations susdites forment un élément d'une pièce (33), en ce que les pièces distales (38-40) de ces articulations forment aussi un élément d'une pièce (40) et en ce que les deux éléments d'une pièce (33, 40) sont articulés l'un sur l'autre par lesdits moyens de guidage.

11. Appareil suivant l'une des revendications 8 à 10, caractérisé en ce que les deux éléments d'une pièce (33, 40) présentent chacun une surface de glissement (43, 44) qui sont capables de glisser l'une sur l'autre pendant le mouvement de l'appareil.

12. Appareil suivant' l'une des revendications 8 et 10, caractérisé en ce que l'élément d'une pièce formé par les pièces proximales est un bloc traversé latéralement par une rainure ouverte de part et d'autre du plan antéro-postérieur, en ce que chacune des pièces distales présente au moins un élément suiveur capable de coulisser dans cette rainure d'un côté du plan antéro-postérieur et en ce que la rainure fournit d'un côté du plan antéro-postérieur une première trajectoire de courbure donnant un mouvement prédéterminé et d'un autre côté du plan antéro-postérieur une deuxième trajectoire de courbure donnant un mouvement prédéterminé, différent de celui de la première trajectoire.

13. Appareil suivant l'une quelconque des revendications 1 à 12, caractérisé en ce qu'il comprend des premiers moyens de liaison et des pièces proximales façonnés d'une pièce et/ou des deuxièmes moyens de liaison et des pièces distales façonnés d'une pièce.

14. Appareil suivant l'une quelconque des revendications 1 à 13, caractérisé en ce que lesdites surfaces de guidage et surfaces d'appui des moyens de guidage présentent des courbures prédéterminées pour obtenir un mouvement de rotation et un mouvement de varus autour d'axes passant par un plan pivot disposé à une certaine distance vers l'intérieur d'un plan central antéro-postérieur divisant le genou.

## Claims

1. A knee support or replacement apparatus comprising
- at least one outer joint and one inner joint which are arranged each side of a vertical, antero-posterior plane and which each comprise at least one proximal member (7, 8; 19, 20; 49; 31-33) and at least one distal member (1, 2; 13, 14; 47, 48; 38-40) articulated together,
- first connection means (25) for connecting together the proximal members of the joints and second connection means (26) for connecting together the distal members of the joints, the connection means (25, 26) effecting mutual positioning of the joints along a common axis,
- attaching means (51, 52) for fixing the proximal members to a femoral part of a lower member and the distal members to a tibial part of said lower member,
- and means of guiding the pieces articulated together, enabling relative movement of flexion, antero-posterior rolling connected with sliding, rotation and/or varus movement between said femoral and tibial parts, the guide means providing the members of each of said joints with a movement different from that of each of the other joints,
- said guide means of each joint comprising at least one guide surface (4, 10, 16, 22; 29, 30) of predetermined curvature in an articulated member and at least one corresponding follower element (5, 11, 17, 23; 45, 46) on the other articulated member, these articulated members of each joint further exhibiting bearing surfaces (3, 9, 15, 21) of predetermined curvature which cooperate during said relative movement between the femoral and tibial parts,
characterized in that said bearing surfaces of the guide means of each joint have mutually different complex curvatures by means of which they are in only partial contact, at at least three points, during said movement between the femoral and tibial parts.

2. An apparatus according to claim 1, wherein one of the articulated members (1, 47, 48) of one of the joints comprises two guide surfaces (29, 30) of predetermined curvature and another of the articulated members (7, 49) of this joint comprises two follower elements (45, 46) each capable of following one of said two guide surfaces (29, 30).

3. An apparatus according to claim 1 or 2, wherein the articulated members of a joint each comprise, at one end, at least one shell (2, 8, 14, 20, 47-49, 36, 37, 41, 42) provided with at least one slot ( 4, 10, 16, 22, 29, 30) and/or at least one follower element (5, 11, 17, 23, 45, 46) capable of sliding in a slot in the other articulated member, and at least one shell (2, 14, 47-48, 36, 37) of one of the articulated members comprises a bearing surface (3, 15) with a first predetermined complex curvature with which a bearing surface (9, 21) exhibiting a second complex curvature, different from the first, of at least one shell (8, 20, 49, 41-42) of the other articulated member may cooperate by said partial contact during said relative movement between the femoral and tibial parts.

4. An apparatus according to claim 3, wherein one of the articulated members of a joint comprises two shells (47, 48) which are arranged facing each other and each provided with a slot (29, 30) and which comprise bearing surfaces facing each other and of predetermined curvature and the other of the articulated members of this joint comprises a shell (49) which is interpolated between the two above-mentioned shells (47, 48) and is provided with two follower elements (45, 46) each penetrating into one of said slots (29, 30), and which has two bearing surfaces of curvature different from the curvatures of the bearing surfaces facing each other, these bearing surfaces each being capable of interacting by the above-mentioned partial contact with the one of said bearing surfaces facing each other of the two shells during said relative movement between the femoral and tibial parts.

5. An apparatus according to anyone of claims 1 to 4, wherein the follower elements (5, 6, 11, 12, 17, 18, 23, 24) simultaneously act as retaining means for the articulated members articulated in partial contact one with the other.

6. An apparatus according to anyone of claims 1 to 5, wherein the above-mentioned vertical anteroposterior plane passes through the knee.

7. An apparatus according to anyone of claims 1 to 4, wherein the above-mentioned vertical anteroposterior plane is arranged on one side of the knee.

8. An apparatus according to anyone of claims 1, 2 and 5, wherein the proximal members of the above-mentioned joints form a one-piece element and in that the distal members of these joints are articulated to the one-piece element by said guide means.

9. An apparatus according to anyone of claims 1, 2 and 5, wherein the distal members of the above-mentioned joints form a one-piece element and in that the proximal members of these joints are articulated to the one-piece element by said guide means.

10. An apparatus according to anyone of claims 1, 2 and 5, wherein the proximal members (31-33) of the above-mentioned joints form a one-piece element (33), the distal members (38-40) of these joints also forming a one-piece element and the two one-piece elements (33, 40) being articulated to each other by said guide means.

11. An apparatus according to anyone of claims 8 to 10, wherein the two one-piece elements each have a sliding surface (43, 44) capable of sliding over each other during movement of the apparatus.

12. An apparatus according to claim 8 or 10, wherein the one-piece element formed by the proximal members is a block passed through laterally by an open slot on each side of the antero-posterior plane, each of the distal members having at least one follower element capable of sliding in this slot on one side of the antero-posterior plane, the slot providing, on one side of the antero-posterior plane, a first trajectory of curvature giving a predetermined movement and, on the other side of the antero-posterior plane, a second trajectory of curvature giving a predetermined movement different from that of the first trajectory.

13. An apparatus according to anyone of claims 1 to 12, comprising first connection means and proximal members made in one piece and/or second connection means and distal members made in one piece.

14. An apparatus according to anyone of claims 1 to 13, wherein said guide surfaces and bearing surfaces of the guide means exhibit predetermined curvatures to obtain a rotational movement and a varus movement about axes passing through a pivot plane arranged at a certain distance towards the inside of a central antero-posterior plane dividing the knee.

## Patentansprüche

1. Apparat zur Unterstützung oder zum Ersatz eines Knies,
- mit wenigstens einem äußeren Gelenk und einem inneren Gelenk, die beidseits einer vertikalen, nach vorne und hinten gerichteten Ebene angeordnet sind und jeweils wenigstens ein proximales Teil (7, 8; 19, 20; 49; 31-33) sowie wenigstens ein distales Teil (1, 2; 13, 14; 47, 48; 38-40) umfasssen, die gegenseitig gelenkig miteinander verbunden sind,
- mit ersten Verbindungsmitteln (25) zur gegenseitigen Verbindung der proximalen Teile der Gelenke und mit zweiten Verbindungsmitteln (26) zur gegenseitigen Verbindung der distalen Teile der Gelenke, wobei die Verbindungsmittel (25, 26) eine gegenseitige Positionierung der Gelenke gemäß einer gemeinsamen Achse (50) realisieren,
- mit Befestigungsmitteln (51, 52) zur Festlegung der proximalen Teile an einem femoralen Abschnitt eines unteren Gliedes und zur Festlegung der distalen Teile an einem tibialen Abschnitt dieses unteren Gliedes, und
- mit Führungsmitteln der gegenseitig gelenkig miteinander verbundenen Teile, welche eine relative Beugungsbewegung, eine einer Gleitbewegung zugeordnete, in einer von vorne nach hinten gerichteten Ebene verlaufende Rollbewegung, eine Rotation und/oder eine Varus-Bewegung zwischen den femoralen und tibialen Abschnitten gestatten, wobei die Führungsmittel den Teilen jedes Gelenkes eine Bewegung erteilen, die von derjenigen eines jeden der anderen Gelenke verschieden ist,
- wobei die Führungsmittel jedes Gelenkes wenigstens eine Führungsfläche (4, 10, 16, 22; 29, 30) vorbestimmter Krümmung in einem Gelenkteil und wenigstens ein entsprechendes Nachführelement (5, 11, 17, 23; 45, 46) am anderen Gelenkteil umfassen, wobei die Gelenkteile jedes Gelenkes weiterhin Abstützflächen (3, 9, 15, 21) vorbestimmter Krümmung aufweisen, die während besagter Relativbewegung zwischen den femoralen und tibialen Abschnitten zusammenwirken,
**dadurch gekennzeichnet,**
daß die genannten Abstützflächen der Führungsmittel jedes Gelenkes komplexe, wechselseitig voneinander verschiedene Krümmungen aufweisen, durch welche sie während der Bewegung zwischen den femoralen und tibialen Abschnitten in einzig und allein teilweisem Kontakt an wenigstens drei Punkten stehen.

2. Apparat nach Anspruch 1, dadurch gekennzeichnet, daß eines der Gelenkteile (1, 47, 48) eines Gelenkes zwei Führungsflächen (29, 30) vorbestimmter Krümmungen aufweist, und daß ein anderes Gelenkteil (7, 49) dieses Gelenks zwei Nachführelemente (45, 46) besitzt, die jeweils befähigt sind, einer der beiden Führungsflächen (29, 30) zu folgen.

3. Vorrichtung nach Anspruch 1 oder 2, dadurch gekennzeichnet, daß die Gelenkteile eines Gelenkes jeweils an einem Ende wenigstens eine Schale (2, 8, 14, 20, 47-49, 36, 37, 41, 42), die mit wenigstens einer Nut (4, 10, 16, 22, 29, 30) versehen sind und/oder wenigstens ein Nachfolgeelement (5, 11, 17, 23, 45, 46) umfassen, das befähigt ist, in einer Nut des anderen Gelenkteils zu gleiten, und daß wenigstens eine Schale (2, 14, 47-48, 36, 37) eines der Gelenkteile eine Abstützfläche (3, 15) umfaßt, welche eine erste komplexe, vorbestimmte Krümmung aufweist, mit welcher eine Abstützfläche (9, 21), welche eine zweite komplexe, von der ersten verschiedene Krümmung besitzt und an wenigstens einer Schale (8, 20, 49, 41-42) des anderen Gelenkteils angeordnet ist, durch den teilweisen Kontakt während der Relativbewegung zwischen den femoralen und tibialen Abschnitten zusammenwirken kann.

4. Apparat nach Anspruch 3, dadurch gekennzeichnet, daß eines der Gelenkteile eines Gelenkes zwei Schalen (47, 48) umfaßt, die einander gegenüberliegend angeordnet und jeweils mit einer Nut (29, 30) versehen sind und die Abstützflächen umfassen, welche sich mit vorbestimmter Krümmung einander gegenüberliegen, und daß das andere Gelenkteil dieses Gelenks eine Schale (49) umfaßt, die zwischen die beiden vorgenannten Schalen (47, 48) eingesetzt und mit zwei Nachführelementen (45, 46) versehen ist, die jeweils in eine der Nuten (29, 30) eindringen, wobei die Schale (49) zwei Abstützflächen mit einer Krümmung aufweist, die von den Krümmungen der sich einander gegenüberliegenden Abstützflächen verschieden ist, und wobei diese Abstützflächen jeweils in der Lage sind, über einen partiellen Kontakt während der Relativbewegung zwischen den femoralen und tibialen Abschnitten mit einer der sich einander gegenüberliegenden Abstützflächen der beiden Schalen zusammenzuwirken.

5. Apparat nach einem der Ansprüche 1 bis 4, dadurch gekennzeichnet, daß die Nachführelemente (5, 6, 11, 12, 17, 18, 23, 24) gleichzeitig als Haltemittel für die Gelenkteile dienen, die miteinander in gelenkigem, teilweisen Kontakt sind.

6. Apparat nach einem der Ansprüche 1 bis 5, dadurch gekennzeichnet, daß die vertikale, von vorne nach hinten gerichtete Ebene das Knie schneidet.

7. Apparat nach einem der Ansprüche 1 bis 4, dadurch gekennzeichnet, daß die vertikale, von vorne nach hinten gerichtete Ebene auf einer Seite des Knies angeordnet ist.

8. Apparat nach einem der Ansprüche 1, 2 und 5, dadurch gekennzeichnet, daß die proximalen Teile der Gelenke ein Element eines Teiles bilden, und daß die distalen Teile dieser Gelenke durch die Führungsmittel auf dem Element eines Teiles angelenkt sind.

9. Apparat nach einem der Ansprüche 1, 2 und 5, dadurch gekennzeichnet, daß die distalen Teile der Gelenke ein Element eines Teiles bilden, und daß die proximalen Teile dieser Gelenke durch die Führungsmittel auf dem Element eines Teiles angelenkt sind.

10. Vorrichtung nach einem der Ansprüche 1, 2 und 5, dadurch gekennzeichnet, daß die proximalen Teile (31-33) der Gelenke ein Element eines Teiles (33) bilden, daß die distalen Teile (38-40) dieser Gelenke ebenfalls ein Element eines Teils (40) bilden, und daß die beiden Elemente eines Teiles (33, 40) durch die Führungsmittel miteinander gelenkig verbunden sind.

11. Apparat nach einem der Ansprüche 8 bis 10, dadurch gekennzeichnet, daß die Elemente eines Teiles (33, 40) jeweils eine Gleitfläche (33, 44) aufweisen, wobei die Gleitflächen befähigt sind, während der Bewegung des Apparates aufeinander zu gleiten.

12. Apparat nach einem der Ansprüche 8 und 10, dadurch gekennzeichnet, daß das Element eines Teiles, das durch die proximalen Teile gebildet ist, ein Block ist, der seitlich von einer Nut durchquert wird, die beidseits der von vorne nach hinten gerichteten Ebene offen ist, daß jedes der distalen Teile wenigstens ein Nachführelement aufweist, welches befähigt ist, in dieser Nut auf einer Seite der von vorne nach hinten gerichteten Ebene zu gleiten, und daß die Nut auf einer Seite der von vorne nach hinten gerichteten Ebene eine erste Krümmungslaufbahn liefert, die eine vorbestimmte Bewegung ergibt, und daß die Nut auf der anderen Seite der von vorne nach hinten gerichteten Ebene eine zweite Krümmungslaufbahn vermittelt, die eine vorbestimmte Bewegung ergibt, welche von derjenigen der ersten Laufbahn verschieden ist.

13. Apparat nach einem der Ansprüche 1 bis 12, dadurch gekennzeichnet, daß er erste Verbindungsmittel und proximale Teile, die nach einem Teil gestaltet sind, und/oder zweite Verbindungsmittel und distale Teile umfaßt, die nach einem Teil gestaltet sind.

14. Vorrichtung nach einem der Ansprüche 1 bis 13, dadurch gekennzeichnet, daß die Führungsflächen und die Abstützflächen der Führungsmittel vorbestimmte Krümmungen aufweisen, um eine Rotationsbewegung und eine Varus-Bewegung um Achsen zu erhalten, welche durch eine Schwenkebene verlaufen, die in einem bestimmten Abstand gegenüber dem Inneren einer zentralen, von vorne nach hinten gerichteten, das Knie teilenden Ebene angeordnet ist.
